# EUROPEAN PATENT SPECIFICATION

(11) **EP 1 153 906 B1**
(45) Date of publication and mention of the grant of the patent: **21.05.2003**
(21) Application number: 01301447.7
(22) Date of filing: 19.02.2001
(51) Int. Cl.: C07C 17/21, C07C 17/383, C07C 19/08

(54) **Preparation of 245fa**
Herstellung von 245fa
Préparation de 245fa

(30) Priority: 08.05.2000 US 567169
(43) Date of publication of application: 14.11.2001
(73) Proprietor: Atofina Chemicals, Inc., Philadelphia, PA 19103-3222 (US)
(72) Inventor: Chen, Bin, Wayne, PA 19087 (US); Elsheikh, Maher Y., Wayne, PA 19087 (US)
(74) Representative: Stoner, Gerard Patrick

(56) References cited:
- EP-A- 0 939 071
- GB-A- 2 313 118
- US-A- 5 877 359
- US-A- 5 981 814

## Description

This invention relates to new ways of preparation of 1,1,1,3,3-pentafluoropropane ("245fa") from 1,1,1-trifluoro-3-chloro-2-propene ("1233zd"). wherein said 1233zd is contacted with hydrogen fluoride ("HF") in the presence of a metal salt catalyst, particularly to improvements in the catalyst lifetime resulting from use as the starting material of 1233zd having less than 100 ppm of olefinic impurities of the formula CF₃₋ₐClₐCH=CH_{b}Cl_{2-b}, where a = 1 to 3 and b = 0 to 2. 1,1,1,3,3-Pentafluoropropane is known to have utility as a foam blowing agent and refrigerant.

The starting material 1233zd is generally produced by fluorinating chloroalkenes such as 1,1,3,3-tetrachloropropene (1230za), such as is taught in U.S. Patent 5,877,359, or chloroalkanes such as 1,1,1,3,3-pentachloropropane (240fa), such as is taught in U.S. Patent 5,710,352, such processes typically resulting in the presence of the aforementioned olefinic impurities as a result of underfluorination. We have found that when hydrofluorinating these 1233zd starting materials such impurities are responsible for deactivation (shortening of the lifetime) of the metal salt catalysts used in such hydrofluorinations.

### BRIEF SUMMARY OF THE INVENTION

In a process for the hydrofluorination of 1233zd to 245fa in the presence of a metal salt catalyst, an improvement is provided which comprises using as the starting material 1233zd having less than 100 ppm of olefinic impurities of the formula CF₃₋ₐClₐCH=CH_{b}Gl_{2-b}, where a is 1, 2 or 3 and b is 0, 1 or 2.

### DETAILED DESCRIPTION

It has now been discovered that use of a 1233zd starting material having a low level of the aforementioned olefinic impurities can give significant improvement in the lifetime of the metal salt hydrofluorination catalyst, as is demonstrated below.

Where the 1233zd starting material contains high levels of these impurities, the 1233zd is pre-treated by conventional separation techniques such as distillation to lower the impurity level to below 100 ppm, preferably to below 10 ppm. At levels of 100 ppm and above the catalyst begins to suffer activity loss. Routine distillation has been found effective to bring olefinic impurities, such as 1,1-difluoro-1,3-dichloro-2-propene and 1-fluoro-1,1,3-trifluoro-2-propene, to levels generally undetectable by gas chromatography, that is, to levels below 10 ppm.

This process is applicable with any effective metal salt catalyst, unsupported or supported, e.g. salts of chromium, nickel, cobalt, iron, copper, zinc, aluminum, lithium, potassium, sodium, magnesium, manganese, antimony, titanium, tin, tantalum, niobium, molybdenum, rhodium and combinations thereof. Preferred catalysts are chromium (III) based, such as a chrome/nickel catalyst on an aluminum fluoride support as taught in European Patent 486333. The salts are typically halides such as chlorides or fluorides. Such metal halide catalysts maybe made by the reaction of metal oxide with HCl or HF. Disclosures of typical catalysts, supports, methods of activation, and the co-use of air or chlorine co-feeds are taught, for example, in U.S. Patent 5,811,603, the disclosure of which is hereby incorporated by reference with respect to its teachings of catalysts and cofeeds.which may all be adopted herein.

The hydrofluorination process temperature is typically between 25°C. and 600°C., preferably between 50°C. and 400°C. The pressure is typically between 15 and 41,4 bar (600 psig), preferably between 50 and 27,6 bar (400 psig). The molar ratio of HF to 1233zd is generally between 2 and 200, preferably between 3 and 50.

The practice of the invention is illustrated in more detail in the following nonlimiting example, using the Cr-Ni/AlF₃ catalyst of European Patent 486333 to hydrofluorinate 1233zd to 245fa:

### Example

Two different feeds were used, (a) 1233zd containing no detectable level of impurities of the above formula, CF₃₋ₐClₐCH=CH_{b}Cl_{2-b}, and (b) 1233zd containing 1600 ppm of the impurity 1,1-difluoro-1,3-dichloro-2-propene. The 1233zd and HF reactants were premixed and passed over the catalyst at a temperature of about 380°C and a pressure of about 10,7 bar (155 psig), using an HF/1233zd molar ratio of about 4:1 and an air co-feed. When reacting the impure 1233zd the catalyst lifetime was found to be 140 hours, while the catalyst used with the pure 1233zd starting material was still active after more than 400 hours.

## Claims

1. A process for the hydrofluorination of 1,1,1-trifluoro-3-chloro-2-propene to 1,1,1,3,3-pentafluoropropane in the presence of a metal salt catalyst, **characterised by** using as the starting material 1,1,1-trifluoro-3-chloro-2-propene having less than 100 ppm of olefinic impurities of the formula CF₃₋ₐClₐCH=CH_{b}Cl_{2-b}, where a is 1, 2 or 3 and b is 0, 1 or 2.

2. The process of Claim 1 wherein the catalyst is chromium-based.

3. The process of Claim 1 or 2 wherein the 1,1,1-trifluoro-3-chloro-2-propene starting material has been pre-treated to reduce the level of said olefinic impurities.

4. A process of claim 3 comprising said pre-treatment as a step.

5. A process according to any one of the preceding claims comprising the preliminary preparation of said starting material by the fluorination of chloroalkene.

## Patentansprüche

1. Verfahren zur Hydrofluorierung von 1,1,1-Trifluor-3-chlor-2-propen zu 1,1,1,3,3-Pentafluorpropan in Gegenwart eines Metallsalzkatalysators, **dadurch gekennzeichnet, dass** als Ausgangsmaterial 1,1,1-Trifluor-3-chlor-2-propen mit weniger als 100 ppm an olefinischen Verunreinigungen der Formel CF₃₋ₐClₐCH=CH_{b}Cl_{2-b}, worin a 1, 2 oder 3 und b 0,1 oder 2 ist, verwendet wird.

2. Verfahren nach Anspruch 1, worin der Katalysator auf Chrom basiert.

3. Verfahren nach Anspruch 1 oder 2, worin das 1,1,1-Trifluor-3-chlor-2-propen-Ausgangsmaterial vorbehandelt worden ist, um den Gehalt an den olefinischen Verunreinigungen zu reduzieren.

4. Verfahren nach Anspruch 3, umfassend die Vorbehandlung als eine Stufe.

5. Verfahren nach einem der vorhergehenden Ansprüche, umfassend die Vorabherstellung des Ausgangsmaterials durch die Fluorierung von Chloralken.

## Revendications

1. Procédé pour la fluorhydratation du 1,1,1-trifluoro-3-chloro-2-propène en 1,1,1,3,3-pentafluoropropane en présence d'un catalyseur à base d'un sel métallique, **caractérisé par** l'utilisation comme matière de départ de 1,1,1-trifluoro-3-chloro-2-propène renfermant moins de 100 ppm d'impuretés oléfiniques, de formule CF₃₋ₐClₐCH=CH_{b}Cl_{2-b}, dans laquelle a est égal à 1, 2 ou 3 et b est égal à 0, 1 ou 2.

2. Procédé suivant la revendication 1, dans lequel le catalyseur est à base de chrome.

3. Procédé suivant la revendication 1 ou la revendication 2, dans lequel la matière de départ consistant en 1,1,1-trifluoro-3-chloro-2-propène a été prétraitée pour réduire la teneur en lesdites impuretés oléfiniques.

4. Procédé suivant la revendication 3, comprenant ledit prétraitement comme étape.

5. Procédé suivant l'une quelconque des revendications précédentes, comprenant la préparation préliminaire de ladite matière de départ par fluoration d'un chloralcène.
